# EUROPEAN PATENT APPLICATION

(11) **EP 4 309 654 A1**
(43) Date of publication of application: **24.01.2024**
(21) Application number: 22382700.7
(22) Date of filing: 21.07.2022
(51) Int. Cl.: A61K 31/404, A61K 31/519, A61K 45/06, A61P 35/00

(54) **INDOLE-3-CARBINOL IN COMBINATION WITH IBRUTINIB FOR THE TREATMENT OF B LYMPHOID NEOPLASMS**

(71) Applicant: Consejo Superior de Investigaciones Científicas (CSIC), 28006 Madrid (ES); Universidad Autónoma de Madrid, 28049 Madrid (ES)
(72) Inventor: ZAPATA HERNÁNDEZ, Juan Manuel, 28006 Madrid (ES); PÉREZ CHACÓN, Gema, 28006 Madrid (ES); BARBERO CASCÓN, Miguel, 28006 Madrid (ES)
(74) Representative: Elzaburu S.L.P.

(57) **Abstract**

The present invention relates to a pharmaceutical combination of indole-3-carbinol or its derivatives with different BCR pathway inhibitors, such as the BTK inhibitors Ibrutinib and zanubrutinib, the TK inhibitor dasatinib or the PI3Kδ inhibitor Idelalisib, for the treatment of B lymphoid neoplasms. The invention also refers to a pharmaceutical composition comprising both types of these compounds and optionally further active compounds.

## Description

### FIELD OF THE INVENTION

The present invention relates to the use of the compound indole-3-carbinol or its derivatives in combination with different BCR pathway inhibitors, such as the BTK inhibitors Ibrutinib and zanubrutinib, the TK inhibitor dasatinib or the Pl3Kδ inhibitor Idelalisib, for the treatment of B lymphoid neoplasms. Therefore, the present invention relates to the field of pharmacology or pharmaceutical chemistry.

### BACKGROUND OF THE INVENTION

Indole-3-carbinol (I3C) [(1H-indol-3-yl)methanol] is a derivative of glucobrassicin (or indole-3-glucosinolate) found in edible plants of the Brassica genus, such as broccoli, cauliflower and cabbages. Several studies have described I3C and its dimer 3,3'-diindolylmethane (DIM) as chemopreventive and antitumour agents. Indeed, these compounds have been shown to inhibit inflammation, cell proliferation and tumour invasion. Phase I and II clinical trials have shown that oral administration of these compounds is effective in promoting regression of precancerous lesions of the cervix, vulvar epidermal neoplasia and recurrent respiratory papillomatosis, without toxic effects in humans at the doses administered.

The pharmacological effect of I3C alone or in combination with fludarabine (chemotherapic drug) in endemic Burkitt's lymphoma (Epstein-Barr virus positive, EBV+) and in cells from patients with chronic lymphocytic leukaemia (CLL) has been described (Perez-Chacon et al., 2014, Pharmacol. Res. 89:46-56; Perez-Chacón et al., 2016, Clin. Cancer Res. 22:134-145). It has been also described that I3C synergised strongly with fludarabine in cells from CLL patients regardless of subtype and grade, and independent of p53 functionality and the existence of multidrug resistance. Indeed, I3C restored fludarabine sensitivity in fludarabine-resistant patient cells (Perez-Chacon et al., 2016, Clin Cancer Res. 22:134-145). Document WO2015044491A1, describes the use of I3C for treating lymphoid neoplasia in combination with fludarabine, a drug used as standard in the treatment of LLC / small cell B lymphoma.

### DESCRIPTION OF THE INVENTION

The present invention identifies indole-3-carbinol as a potential adjuvant or adjuvant drug for treatments with different BCR pathway inhibitors, including the BTK inhibitors Ibrutinib and zanubrutinib, the TK inhibitor dasatinib and the PI3Kδ inhibitor Idelalisib, in both chronic lymphocytic leukaemia patient cells and endemic Burkitt's lymphoma cell lines (EBV+). In addition, the dose of I3C where synergies with these treatments occur is within the bioavailability of this drug (22 µM in blood). I3C synergises with Ibrutinib in combination treatments even in conditions that recapitulate the tumour environment of a B neoplasm, such as the culture of cells from CLL patients on mesenchymal cells or in hypoxia (CLL and Burkitt's lymphoma lines). Proteomic analysis of the effect of the combined treatment of I3C +ibrutinib shows that it enhances the inhibitory effect of the expression of anti-apoptotic proteins (clAP, XIAP, MCL1, cMyc) and increases apoptosis (PARP cleavage). The effect of I3C as an adjuvant in combination therapy with BCR pathway inhibitors is not observed with other drugs also used in the treatment of various B neoplasms (doxorubicin and methotrexate) and other solid tumours (lapatinib, vemurafenib, 5-Fluorouracil, abraxane). I3C and Ibrutinib maintain the cooperative anti-tumour effect in in vivo treatments in mice xenotransplanted with Burkitt's lymphoma cell line BL60.2.

I3C in combined treatment with any of the inhibitors of the BCR pathway (BTK inhibitors, TK inhibitors or Pl3Kδ inhibitors) could represent a significant advance in the therapy of different types of B neoplasms as I3C potentiates the effect of the other drugs, which 1) would increase their activity, could reduce treatment cycles, reduce drug doses, minimise the side effects of the treatments and could reduce or delay the development of relapses, which are very frequent in these tumours.

Thus, in a first aspect the present invention refers to a pharmaceutical combination comprising:
a) a compound of formula (I) 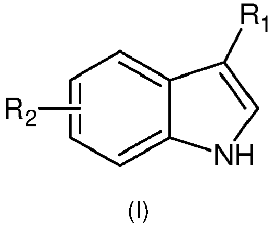 or its derivatives, pharmaceutical acceptable salts or solvates, wherein
   R¹ represents an atom other than hydrogen, of electronegative nature selected from -NH₂, -OH, -SH, -PH₂ , -CI, -Br, -F, -I, -OR, -NR₂ , -SR, - PR₂ , -CH₂OH, -CH₂NH₂ , -CH₂SH, - CH₂PH₂, -CH₂Cl, -CH₂Br, -CH₂F and -CH₂I, and R₂ represents a substituent in position 5 or 6 of the indole ring selected from hydrogen, a C₁-C₆ alkyl group, a halogen atom selected from chlorine, bromine, fluorine or iodine, or an electronegative atom linked to alkyl chains selected from -OR, -NR₂, -SR and PR₂;
   where each R is independently an alkyl group, linear or branched and
b) an inhibitor of the BCR pathway selected from ibrutinib, acalabrutinib, zanubrutinib, evobrutinib, tirabrutinib, vecabrutinib, dasatinib, idelalisib, copanlisib, duvelisib, gedatolisib, buparlisib, alpelisib, taselisib or rigosertib.

In one embodiment, the derivative of the compound of formula (I) is a compound of formula (II): 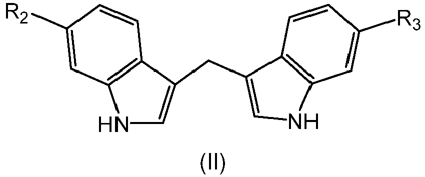 wherein R₂ and R₃ are the same or different substituents and represent hydrogen, an alkyl group, a halogen atom selected from chlorine, bromine, fluorine or iodine, or an electronegative atom attached to alkyl chains selected from -OR, -NR₂, -SR and PR₂, where each R is independently an alkyl group, linear or branched.

In another embodiment, the derivative of the compound of formula (I) is a compound of formula (III): 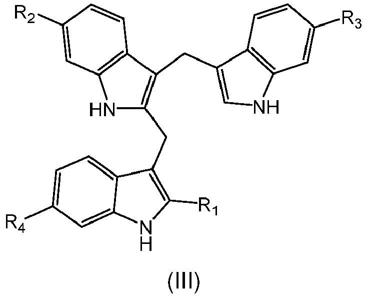 wherein R₁ is as defined in claim 1 and, R₂, R₃ and R₄ are the same or different substituents and represent hydrogen, an alkyl group, a halogen atom selected from chlorine, bromine, fluorine or iodine, or an electronegative atom attached to alkyl chains selected from -OR, -NR₂, -SR and PR₂, where each R is independently an alkyl group, linear or branched.

In a more preferred embodiment, the compound of formula (I) is selected from the following compounds:

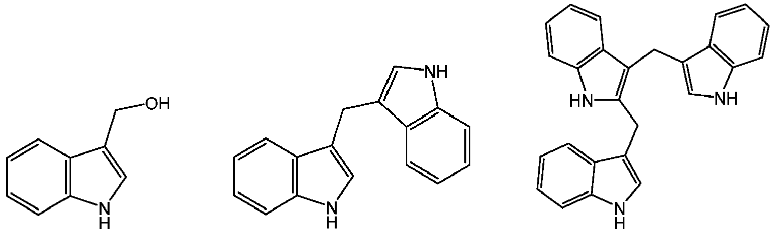

In another embodiment, the inhibitor or the BCR pathway is ibrutinib.

In a more preferred embodiment, the compound of formula (I) is indole-3-carbinol and the inhibitor of the BCR pathway is ibrutinib.

In a preferred embodiment, the pharmaceutical combination described above also comprises c) a further active an adenine analog, selected from 9-β-D-arabinosyl-2-fluoroadenine-5'-monophosphate (F-ara-AMP, fludarabine) or 2-chloro-2'-deoxyadenosine (CdA, cladribine).

In a more preferred embodiment, the pharmaceutical combination described above also comprises c) fludarabine.

Another aspect of the invention refers to a pharmaceutical combination as described above for use in the treatment of B lymphoid neoplasms, preferably selected from chronic lymphocytic leukaemia, small cell lymphocytic lymphoma, Burkitt's lymphoma, diffuse large B-cell lymphoma, lymphoplasmacytic lymphoma, mantle cells lymphoma, Waldenstrom's macroglobulinemia, diffuse large B-cell lymphoma, follicular lymphoma, marginal zone lymphoma or multiple myeloma.

In a preferred embodiment, in the pharmaceutical combination of the invention the use of components (a), (b) and (c) may be simultaneous, separate or sequential.

In another preferred embodiment, in the pharmaceutical combination of the invention the use of components (a), (b) and (c) may be by oral or intravenous administration.

The term "combination" or "pharmaceutical combination" as used herein defines either a fixed combination in one dosage unit form or a kit of parts for the combined administration where the therapeutic agents, e.g. the compound of formula (I), the inhibitor of the BCR pathway and the optional active principle, may be administered independently at the same time or separately within time intervals that allow that the therapeutic agents show a cooperative, e.g., synergistic, effect.

As used herein, the term "alkyl group" means a saturated, monovalent unbranched or branched hydrocarbon chain. Examples of alkyl groups include, but are not limited to, (C₁-C₆)alkyl groups, such as methyl, ethyl, propyl, isopropyl, 2-methyl-1-propyl, 2-methyl-1-butyl, 2,2-dimethyl-1-propyl, 2-methyl-1-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, and hexyl. An alkyl group can be unsubstituted or substituted with one or two suitable substituents.

A "pharmaceutically acceptable salt", as used herein, unless otherwise indicated, includes salts of acidic and basic groups which may be present in the compounds of the present invention. Such salts can be prepared in situ during the final isolation and purification of the compounds, or by separately reacting the base or acid functions with a suitable organic or inorganic acid or base, respectively. Suitable salts of the compound include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2 hydroxyethanesulfonate, lactate, maleate, methanesulfonate, nicotinate, 2 naphth- alenesulfonate, oxalate, pamoate, pectinate, persulfate, 3 phenylproionate, picrate, pivalate, propionate, succinate, sulfate, tartrate, thiocyanate, p toluenesulfonate, and undecanoate. Also, the basic nitrogen- containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others.

Another aspect of the invention refers to a pharmaceutical composition comprising the pharmaceutical combination as describe above and at least one acceptable carrier.

Another aspect of the invention refers to a pharmaceutical composition as described above for use in the treatment of B lymphoid neoplasms, preferably selected from chronic lymphocytic leukaemia, small cell lymphocytic lymphoma, Burkitt's lymphoma, diffuse large B-cell lymphoma, lymphoplasmacytic lymphoma, mantle cells lymphoma, Waldenstrom's macroglobulinemia, diffuse large B-cell lymphoma, follicular lymphoma, marginal zone lymphoma or multiple myeloma.

In a preferred embodiment, the pharmaceutical composition for use as described also comprises a further active principle which is fludarabine.

In the pharmaceutical composition of the invention, the compound of formula (I) and the inhibitor of the BCR pathway, and the optional further active principle, may be combined with an excipient, adjuvant and/or pharmaceutically acceptable carrier and formulated for proper administration. Appropriate formulations are, but are not limited to, solid forms (capsules, tablets, pills, tablets, etc.), semi-solid forms (powders, granulated forms, gels or hydrogels, creams, ointments, balsams, mousses, ointments, foams, lotions, etc.) or liquid forms (solutions, suspensions, emulsions, oils, liniments, syrups, serums, vaporizers, aerosols, etc.), or the compound and the inhibitor may be included in a sustained release system. Depending on its formulation, the composition of the invention may be administered orally, intravenously, subcutaneously, intramuscularly or by inhalation, preferably by oral or intravenous administration.

A further aspect of the invention is a method of treatment of B lymphoid neoplasms such as those previously mentioned which comprises administering a therapeutically effective amount of the compound of formula (I) as described above in combination with and an inhibitor of the BCR pathway, and optionally a further active principle, in a patient in need thereof. The inhibitor of the BCR pathway and the optional further active principle may be administered simultaneously, separately or sequentially with the compound of formula (I).

As used herein, the term "effective amount" refers to a quantity of compound of formula (I) sufficient to achieve a desired therapeutic and/or prophylactic effect, e.g.: an amount which results in the prevention of, or a decrease in, one or more symptoms associated with lymphoid neoplasm or an amount necessary to achieve the desired therapeutic effect, which is an improvement, inhibition, mitigation or control of the presence, prevalence, severity, symptoms, etc. of the disease. In the context of therapeutic or prophylactic applications, the amount of a compound administered to the subject will depend on the type and severity of the disease and on the characteristics of the individual, such as general health, age, sex, body weight and tolerance to drugs. It will also depend on the degree, severity and type of disease. The dosing regimen should be adjusted to provide the optimal therapeutic response. The skilled artisan will be able to determine appropriate dosages depending on these and other factors. The therapeutically effective amount is also that in which any toxic or adverse effects are more than compensated for by the beneficial therapeutic effect.

### BRIEF DESCRIPTION OF FIGURES

Figure 1: Effect of the combined treatment of I3C and ibrutinib on CLL cell viability
Figure 2: Synergistic effect of the combined treatment of I3C and ibrutinib
Figure 3: Synergistic effect of the combined treatment of I3C and ibrutinib in CLL cells under hypoxia
Figure 4: Synergistic effect of the I3C and Ibrutinib combined treatment on CLL cells in the presence of stromal cells.
Figure 5: The combined treatment of I3C and ibrutinib induced apoptosis in CLL cells cultured on a monolayer of stromal cells.

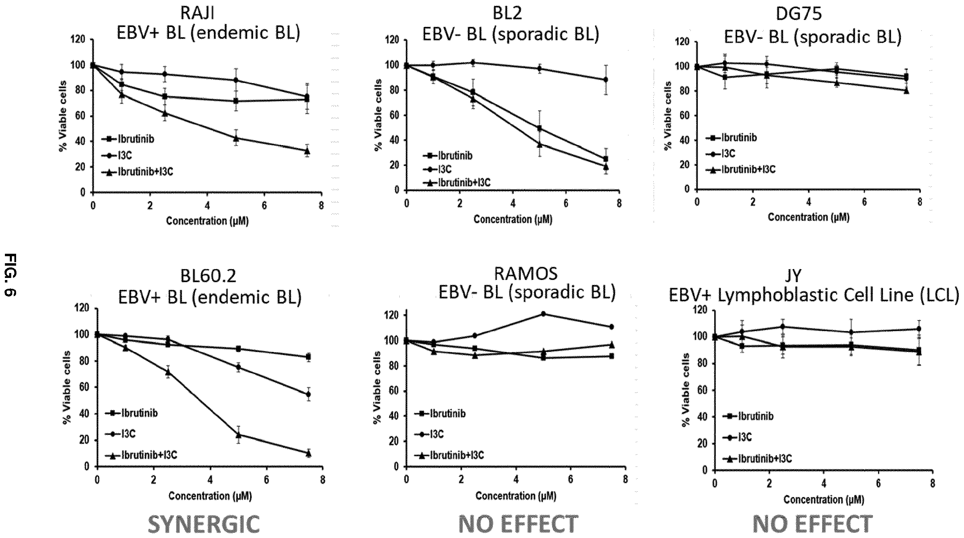

Figure 6: Synergistic effect of the I3C and Ibrutinib combination treatment in EBV⁺ BL Raji cell line.
Figure 7: Synergistic effect of the I3C and Ibrutinib combination treatment in BL60.2 BL cells under hypoxia.
Figure 8: Effect of the combined treatment of I3C and ibrutinib on the expression of proteins involved in the control of apoptosis in CLL cells
Figure 9: Effect of I3C, ibrutinib and combined I3C + ibrutinib treatment on tumour growth in xenotransplanted mice.
Figure 10: Effect of the I3C combined with methotrexate or doxorubicin on the viability of BL cells.
Figure 11: Effect of I3C in combination with a variety of chemotherapeutic drugs used in clinics on the viability different types of solid tumours.
Figure 12: Effect of the combined treatment of I3C with other chemotherapeutics on the viability of CLL cells.
Figure 13: Effect of the combined treatment of I3C with other chemotherapeutics on the viability of BL60.2 cells.

### EXAMPLES

### Example 1: effect of the combined treatment of I3C and ibrutinib on the viability of chronic lymphocytic leukaemia cells.

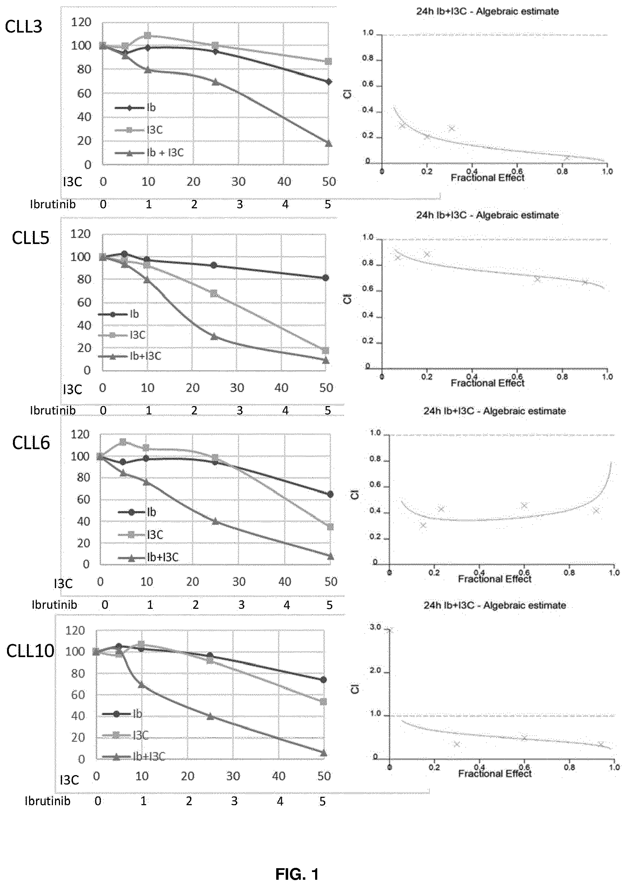

CLL cells (1.5x10⁶ cells/mL) from 6 CLL patients were cultured in triplicates in 96-well microplates. CLL cells were treated with vehicle alone (0.075% ethanol, 0.015% DMSO) or with increasing doses of I3C (5, 10, 25 and 50 µM) (indicated in figure 1), ibrutinib (0.5, 1, 2.5 and 5 µM) or a combination of both compounds, keeping a constant ratio of 10:1 (I3C:ibrutinib). After 48 hours, the percentage of viable cells was determined by a luminometric assay considering the control value as 100% viability. Plots at the right of Fig. 1 show the graphic representation of the combination index (CI) for the fraction affected (Fa) for each group, calculated with the Calcusyn software (CI index <1 = synergy).

### Example 2: synergistic effect of the combined treatment of I3C and ibrutinib.

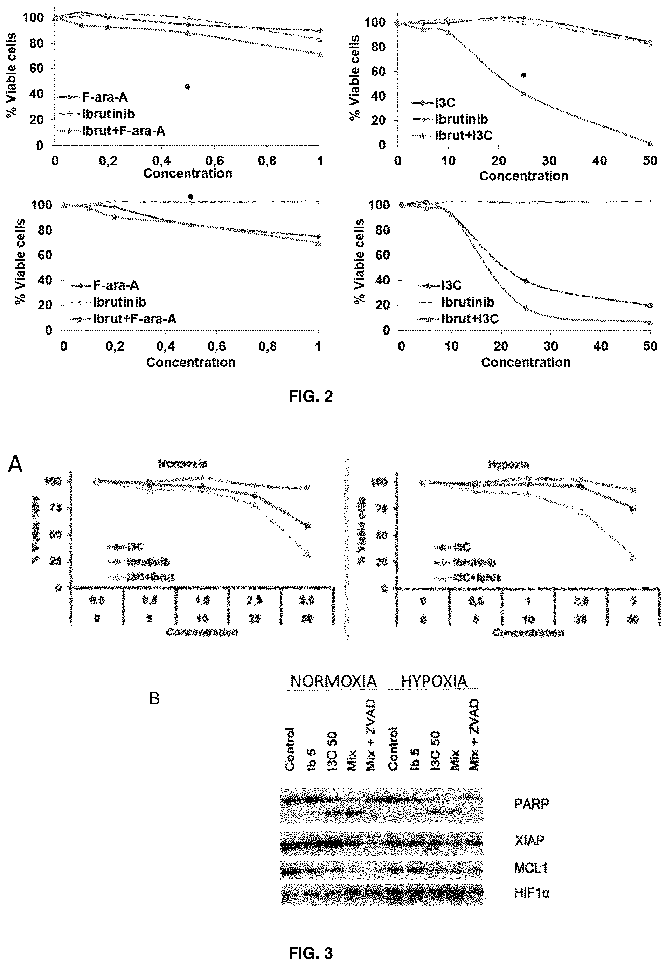

Combination treatment of I3C and ibrutinib, but not Ibrutinib and fludarabine, show potent synergistic effect against PBMCs from CLL patients that are not sensitive to ibrutinib *ex vivo.* CLL cells (1.5x10⁶ cells/mL) from 2 CLL patients having little sensitivity to ibrutinib *ex vivo* were cultured in triplicates in 96-well microplates. CLL cells were treated with vehicle alone (0.075% ethanol, 0.015% DMSO) or with increasing doses of I3C (5, 10, 25 and 50 µM) (indicated in Fig. 2), ibrutinib (0.5, 1, 2.5 and 5 µM) or a combination of both compounds, keeping a constant ratio of 10:1 (I3C:ibrutinib) (right panels of Fig. 2) or with fludarabine (0.1, 0.2, 0.5 and 1) (shown in Fig. 2), ibrutinib (0.5, 1, 2.5 and 5 µM) or a combination of both compounds, keeping a constant ratio of 5:1 (fludarabine:ibrutinib) (left panels). After 48 hours, the percentage of viable cells was determined by a luminometric assay considering the control value as 100% viability.

### Example 3: Synergistic effect of the combination treatment of I3C and ibrutinib under hypoxia in CLL cells.

Combination treatment of I3C and ibrutinib shows potent synergistic effect against CLL cell under hypoxic conditions. CLL cells (1.5x10⁶ cells/mL) from a representative CLL patient were cultured under normoxia (21% O₂ atmosphere) or hypoxia (1-3% O₂ atmosphere). CLL cells were treated with vehicle alone (0.075% ethanol, 0.015% DMSO) or with increasing doses of I3C (5, 10, 25 and 50 µM), ibrutinib (0.5, 1, 2.5 and 5 µM) or a combination of both compounds, keeping a constant ratio of 10:1 (I3C:ibrutinib). After 24 hours, the percentage of viable cells was determined by a luminometric assay considering the control value as 100% viability. See results in Fig. 3A. Combination index (CI) for the fraction affected (Fa) for various drugs combination in normoxia and hypoxia was calculated with the Calcusyn software. Results are shown in the following table:

**Table 1**

| | | ED50 | ED75 | ED90 |
|---|---|---|---|---|
| Normoxia | F-ara-A + I3C | 0.39 | 0.32 | 0.26 |
| | F-ara-A + Ibrutinib | 13.11 | 54.00 | 224.00** |
| | I3C + Ibrutinib | 0.63 | 0.61 | 0.60* |
| | F-ara-A + I3C | 0.21 | 0.18 | 0.15 |
| Hypoxia | F-ara-A + Ibrutinib | 0.83 | 1.64 | 3.22** |
| | I3C + Ibrutinib | 0.13 | 0.09 | 0.01 * |

Values with * indicate that I3C+ibrutinib were synergic under normoxia and hypoxia conditions (CI index <1 = synergy), but fludarabine +ibrutinib were not significant (values with **). In Fig 3B it is shown the effect of the I3C + ibrutinib treatment on the expression and integrity of key survival proteins under normoxia and hypoxia. Cells were treated with the indicated drugs and 24h later, cells were collected, lysed and subjected to SDS-PAGE and immunoblotting. The results show that XIAP and MCL1 are similarly downmodulated and PARP cleaved by the combination treatment in normoxia and hypoxia. As expected, HIFα expression is upregulated under hipoxia.

### Example 4: synergistic effect of the I3C and Ibrutinib combination treatment on CLL cells in the presence of stromal cells.

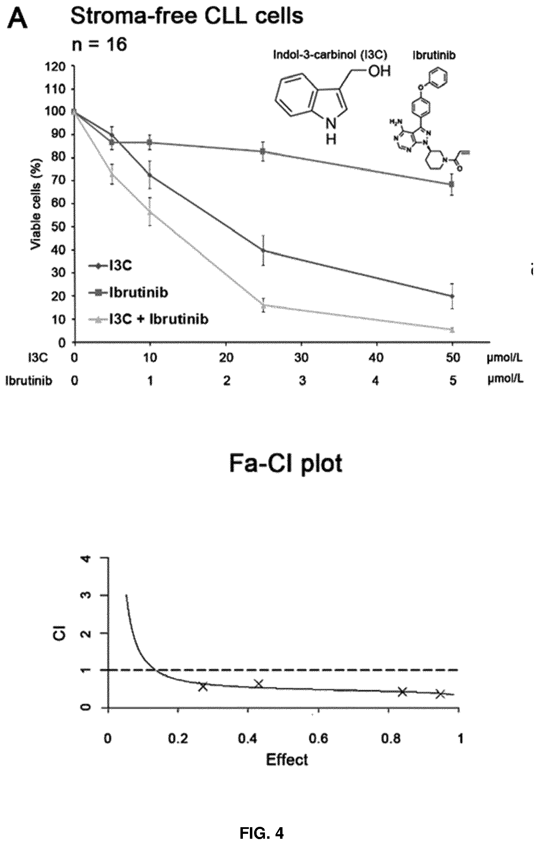

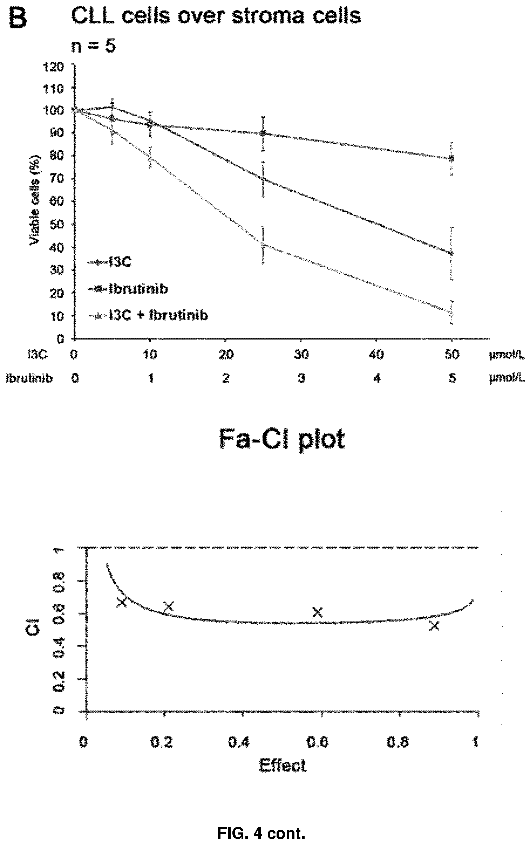

I3C and ibrutinib show synergy against CLL cells and efficiently overcome stroma-mediated drug resistance. CLL cells (1.5x10⁶ cells/mL) were cultured in triplicates in 96-well microplates in the absence (Fig. 4A) or the presence (Fig. 4B) of a monolayer of hMCS-TERT cells (3x10⁴ cells/mL), seeded on the plate 24 hours before the addition of CLL cells. CLL cells were treated with vehicle alone (0.075% ethanol, 0.015% DMSO) or with increasing doses of I3C (5, 10, 25 and 50 µM), ibrutinib (0.5, 1, 2.5 and 5 µM) or a combination of both compounds, keeping a constant ratio of 10:1 (I3C:ibrutinib). After 48 hours, the percentage of viable cells was determined by a luminometric assay (top plot) or by annexin V/PI staining and subsequent flow cytometry (bottom plot), considering the control value as 100% viability. Figures 4A and 4B show mean ± SEM and the number of independent assays (n) is indicated. The structural formulas of I3C and ibrutinib are also shown. Plots at the right show the graphic representation of the combination index (CI) for the fraction affected (Fa) for each group, calculated with the Calcusyn software. Cells from 5 CLL patients were assayed in the absence or in the presence of stroma cells during this work. Data from 11 CLL patients that were tested for the effect of ibrutinib and I3C in the absence of monolayer were already available in the lab and were included in the final analysis (n=16; panel A).

### Example 5: The combined treatment of I3C and ibrutinib induced apoptosis in CLL cells cultured on a monolayer of mesenchymal cells.

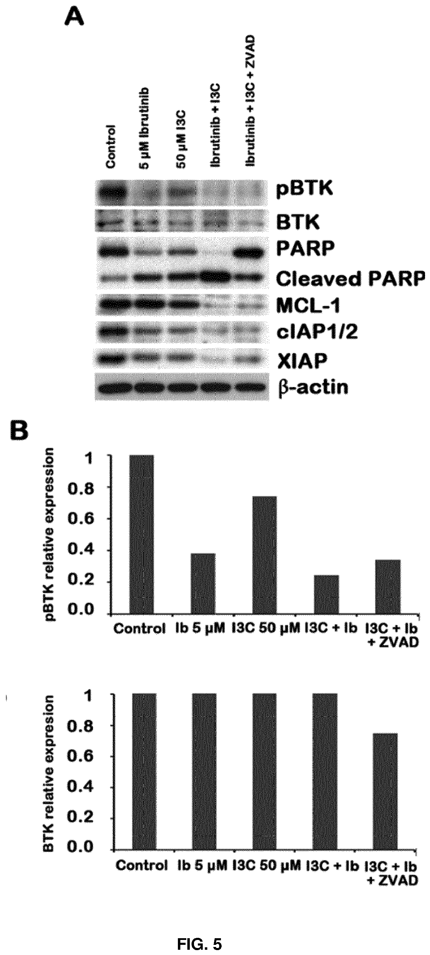

The combined treatment of I3C and ibrutinib induces apoptosis (increases the PARP breakage) and reduces the expression of the antiapoptotic proteins MCL1, cIAP1/2 Y XIAP. PBMCs isolated from a CLL patient (3-4x10⁶ cells/ml) were incubated in 6-well microplates in presence of a monolayer of hMSC-TERT cells (3x10⁴ cell/ml). Next, CLL cells were treated with vehicle (0.075% ethanol, 0.015% DMSO), 5µM ibrutinib, 50µM I3C or the combination of both compounds. When indicated, 100µM Z-VAD-fmk (Z-VAD) was added to the cells for 30 minutes before the drug treatments were initiated. After 48h, cells were lysed in Laemmli buffer and sonicated. In all cases, from 10 to 20 µg of proteins was subjected to 8% SDS-PAGE, followed by immunoblotting with specific antibodies for the proteins of interest. β-actin expression was used as a loading control. Results are shown in Fig.5A. Densitometry analysis of the effects of I3C and/or ibrutinib on the expression of pBTK and BTK shown in Fig. 5A, performed on scanned images using Quantity One software. Values for pBTK and BTK were normalized for BTK and β-actin, respectively, assigning a value of 1 to the control (vehicle) treatment. Results are shown in Fig. 5B.

### Example 6: synergistic effect of the I3C and Ibrutinib combination treatment in EBV⁺ BL Raii cell line.

I3C synergizes with ibrutinib in EBV⁺ BL Raji cell line but not in EBV⁻ BL BL-2 and DG-75 cell lines nor in EBV-infected LCLs. EBV⁺ BL Raji, EBV⁻ BL BL-2, EBV⁻ DG-75 and EBV-infected LCL JY cells lines (1x1 0⁶ cells/mL), were cultured in triplicates in 96-well microplates and treated either with vehicle alone (0.075% ethanol, 0.015% DMSO) or with increasing doses of I3C (10, 25, 50 and 75 µM), ibrutinib (1, 2.5, 5 and 7.5 µM) or a combination of both compounds, keeping a constant ratio of 10:1 (I3C:ibrutinib). After 48 hours, the percentage of viable cells was determined by a colorimetric assay. The result obtained with the cells treated with vehicle was considered as the 100% viability value. Figures show mean ± SEM is indicated. Plots at the right show the graphic representation of the combination index (CI) for the fraction affected (Fa) for each group, calculated with the Calcusyn software. Values in bold and grey are the results showing synergistic effects.

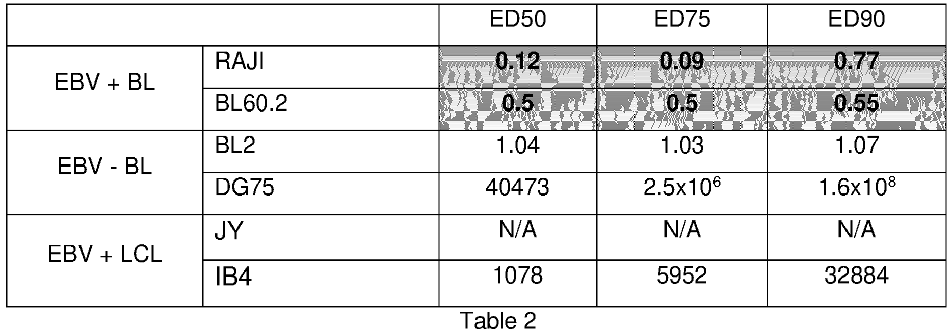

### Example 7: synergistic effect of the I3C and Ibrutinib combination treatment under hypoxia in BL60.2 BL cells.

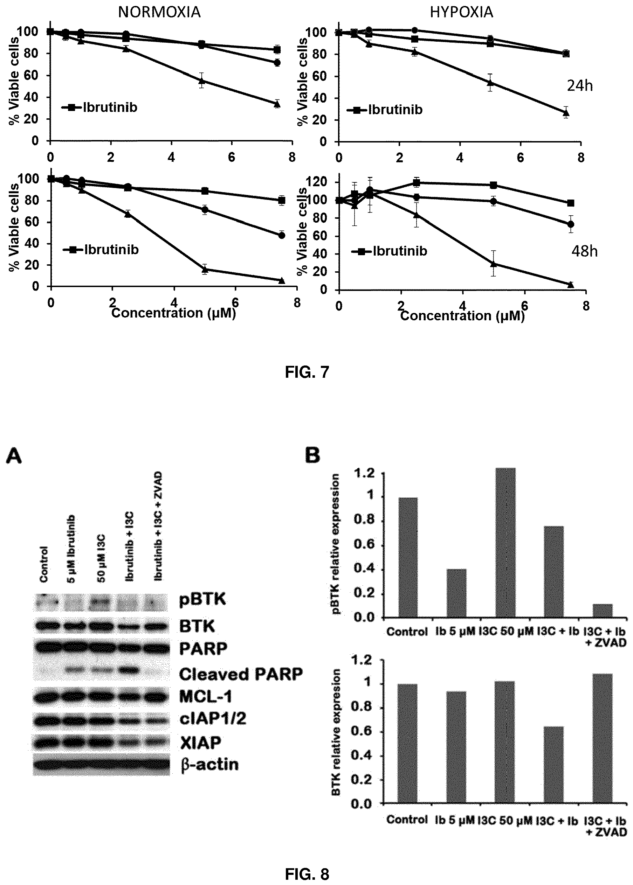

Combination treatment of I3C and ibrutinib shows potent synergistic effect in BL60.2 BL EBV+ cell line cell under hypoxic conditions. BL60.2 cells (10⁶ cells/mL) were cultures under normoxia (21% O₂ atmosphere) or hypoxia (1-3% O₂ atmosphere). BL60.2 cells were treated with vehicle alone (0.075% ethanol, 0.015% DMSO) or with increasing doses of I3C (5, 10, 25 and 50 µM), ibrutinib (0.5, 1, 2.5 and 5 µM) or a combination of both compounds, keeping a constant ratio of 10:1 (I3C:ibrutinib). After 24 and 48 hours, the percentage of viable cells was determined by a luminometric assay considering the control value as 100% viability. Results are shown in Fig. 7A. Combination index (CI) for the fraction affected (Fa) for various drugs combination in normoxia and hypoxia calculated with the Calcusyn software. I3C+ibrutinib were synergic under normoxia and hypoxia conditions (CI index <1 = synergy), as shown in the following table:

**Table 3**

| | | ED50 | ED75 | ED90 |
|---|---|---|---|---|
| Normoxia | 24h | 0.695 | 0.797 | 0.957 |
| | 48h | 0.493 | 0.51 | 0.546 |
| Hypoxia | 24h | 0.58 | 0.59 | 0.545 |
| | 48h | 0.27 | 0.228 | 0.19 |

### Example 8: effect of the combined treatment of I3C and ibrutinib the expression of proteins involved in the control of apoptosis in CLL cells.

Analysis of the effect of I3C, ibrutinib and the combined treatment on the expression of different proteins implicated in apoptosis regulation and BCR signaling pathway in EBV⁺ BL Raji cells was tested. EBV⁺ BL Raji cells (2x10⁶ cells/mL) were incubated in 6-well microplate in medium with vehicle (0.075% ethanol, 0.015% DMSO), 5 µM ibrutinib, 50 µM I3C or the combination of both compounds. When indicated, 100 µM Z-VAD-fmk (Z-VAD) was added to the cells for 30 minutes before the drug treatments were initiated. After 48 hours, cells were lysed in Laemmli buffer and sonicated. In all cases, from 10 to 20 µg of proteins were subjected to 8% SDS-PAGE, followed by immunoblotting with antibodies specific for the proteins of interest. β-Actin expression was used as a loading control. Densitometry analysis of the effects of I3C and/or ibrutinib on the expression of pBTK and BTK is shown in Fig. 8A, performed on scanned images using Quantity One software. Values for pBTK and BTK were normalized for BTK and β-actin, respectively, assigning a value of 1 to the control (vehicle) treatment (Fig. 8B).

### Example 9: Effect of 13C, ibrutinib and combined I3C + ibrutinib treatment on tumour growth in xenotransplanted mice.

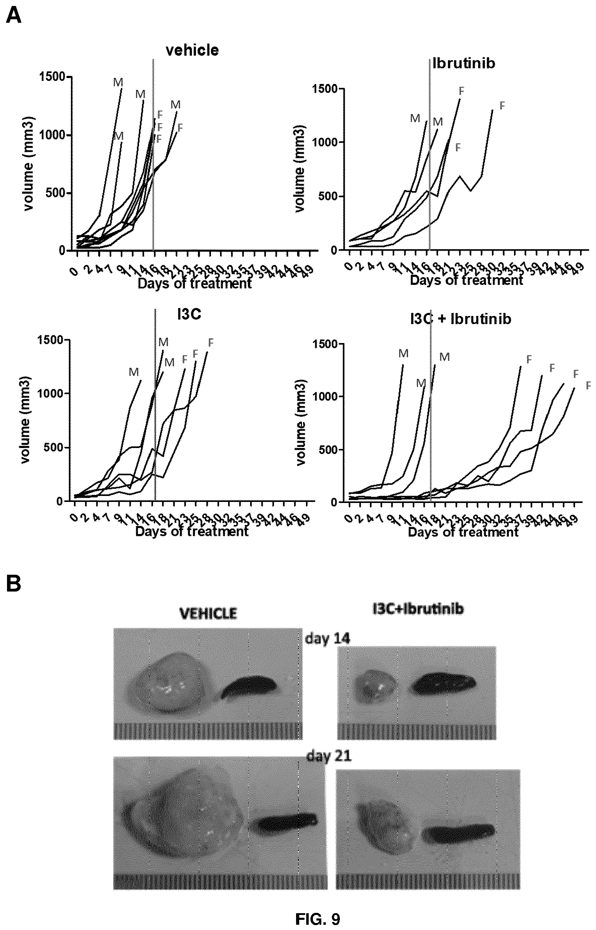

SCID-NOD mice were inoculated on the back with 3 x 106 BL60 cells on matrigel. When tumours were palpable (4-5 mm) mice were treated by gastric gavage from Monday to Friday with vehicle (DMSO, 0.85 ml/kg), I3C (250 mg/kg; 750 mg/m2; dissolved in DMSO), ibrutinib (25 mg/kg ;75 mg/m2; dissolved in DMSO) or combined treatment of 13C+ibrutinib, administered in corn oil (150-175 µl, 20-25g mice). Tumours were measured every 2 days and tumour volume was calculated using the formula d1xd2xd3/2. Mice with tumours > 1 cm3 were sacrificed. Fig. 9A represents tumour size (mm3) as a function of time (days). Fig. 9B shows representative examples of tumours from mice treated with vehicle and the combined I3C+ibrutinib treatment that were sacrificed for analysis at day 14 and 21 of treatment initiation.

### Example 10: Effect of the drugs methotrexate and doxorubicin individually and in combination with I3C on the viability of BL EBV+ Raii and BL EBV- Ramos cells.

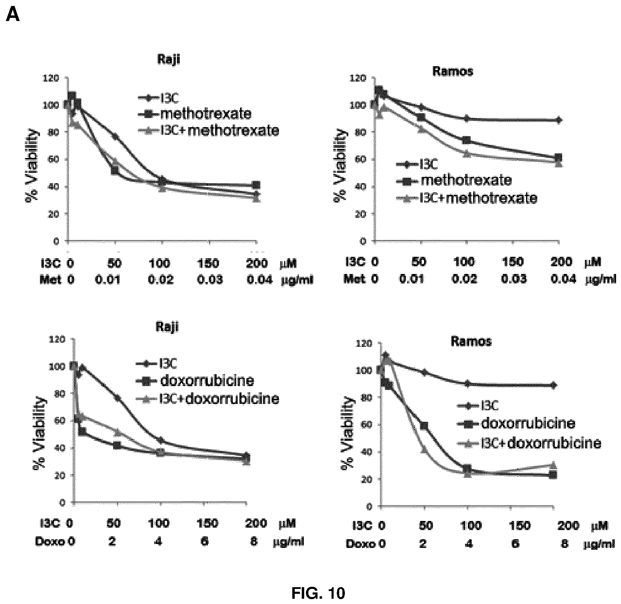

Raji and Ramos cells were cultured in triplicates containing only medium (control condition) and increasing doses of I3C (0, 5, 10, 50, 50, 100 and 200 µM), methotrexate (0.001, 0.002, 0.01, 0.02 and 0. 04 µg/ml) or a combination of both compounds, maintaining a constant ratio of 5000:1 (I3C: methotrexate). Results are shown in Fig. 10A. The same assay was carried out with I3C (0, 5, 10, 50, 50, 100 and 200 µM), doxorubicin (0.2, 0.4, 2, 4 and 8 µg/ml) or a combination of both, maintaining a constant ratio of 25:1 (I3C:doxorubicin). Results shown in Fig. 10B. At 48h, the percentage of viable cells was determined considering the control as 100% viable.

### Example 11: effect of I3C in combination with a variety of chemotherapeutic drugs used in clinics on the viability of different types of solid tumours.

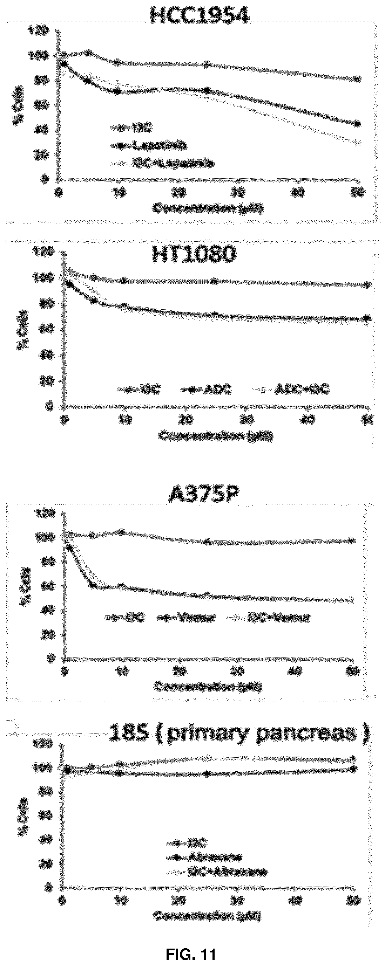

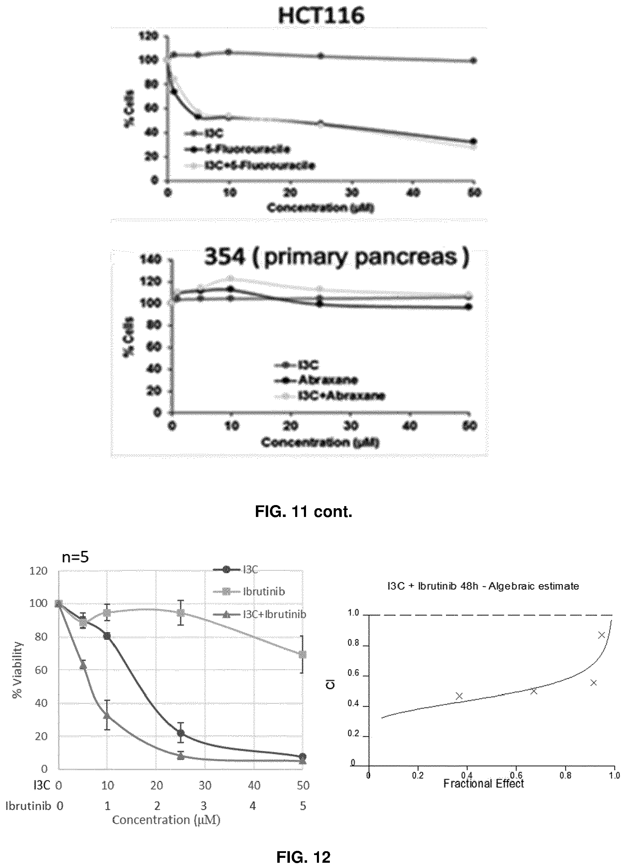

We studied the effect of I3C alone or in combination with different drugs commonly used in the clinic for the targeted treatment of solid tumours of various origins. We used the ductal breast cancer line HCC1954 and the drug Lapatinib, the melanoma line A375P and the drug Vemurafenib, the colorectal cancer line HCT116 and the drug 5-fluorouracil, the sarcoma line HT1080 and the experimental drug ADC, and two primary pancreatic cancer lines 185 and 354 and the drug Abraxane. Results obtained (Fig. 11) shown that I3C has no effect on different types of solid tumours.

### Example 12: Effect of the combination treatment of I3C with other chemotherapeutics on the viability of CLL cells.

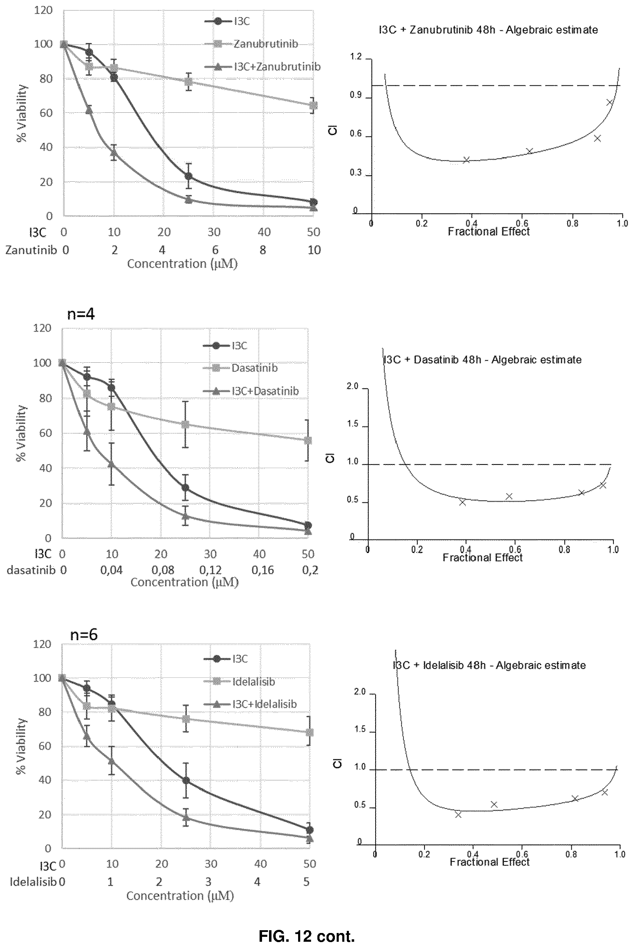

The BTK inhibitors ibrutinib or zanubrutinib, the TK inhibitor dasatinib or the Pl3Kδ inhibitor idelalisib were tested on CLL cells. CLL cells (10⁶ cells/mL) were treated with vehicle alone (0.075% ethanol, 0.015% DMSO) or with increasing doses of I3C (5, 10, 25 and 50µM), as well as with ibrutinib (0.5, 1, 2.5 and 5 µM) (n=5), zanubrutinib (1, 2, 5 and 10µM) (n=4), dasatinib (0.02, 0.04, 0.1 and 0.25 µM) (n=4) and idelalisib (0.5, 1, 2.5 and 5 µM) (n=6), either alone or in combination with I3C, as indicated in the Fig. 12. After 48 hours, the percentage of viable cells was determined by a luminometric assay considering the control value as 100% viability (left). Plots at the right show the graphic representation of the combination index (CI) for the fraction affected (Fa) for each treatment group, calculated with the Calcusyn software.

### Example 13: Effect of the combination treatment of I3C with other chemotherapeutics on the viability of BL60.2 cells.

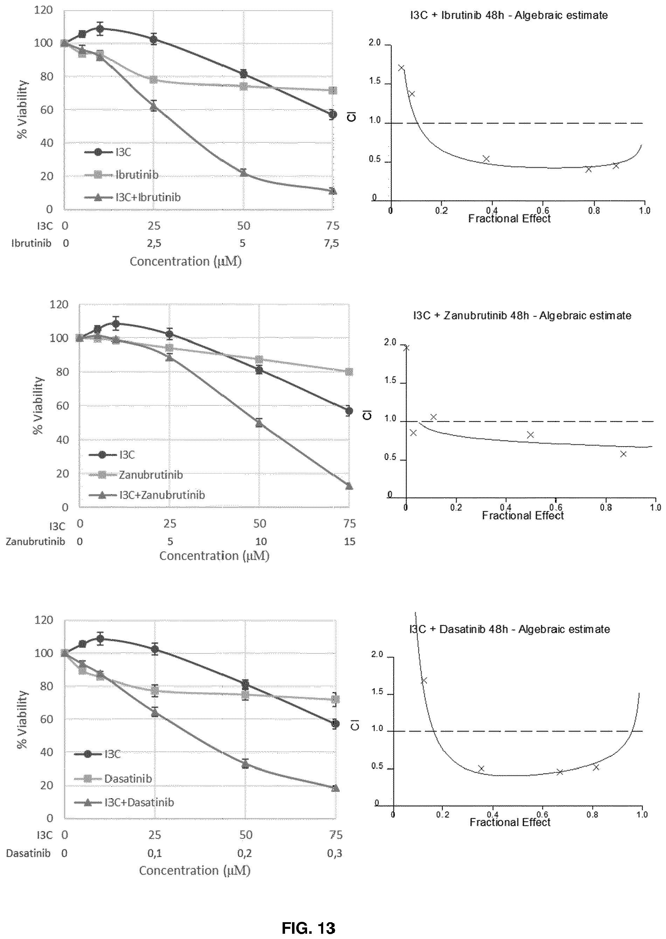

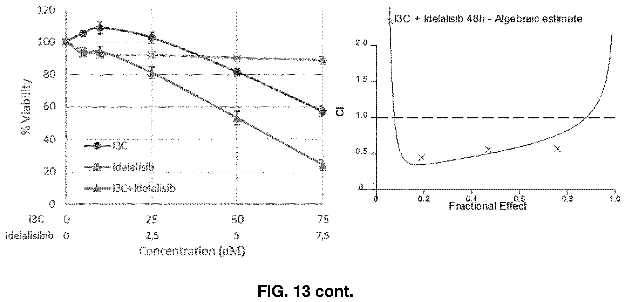

The BTK inhibitors Ibrutinib or zanubrutinib, the TK inhibitor dasatinib and the Pl3Kδ inhibitor idelalisib were tested on BL60.2 Burkitt's lymphoma cells. BL60.2 cells (10⁶ cells/mL) were treated with vehicle alone (0.075% ethanol, 0.015% DMSO) or with increasing doses of I3C (5, 10, 25, 50 and 75µM), as well as with ibrutinib (0.5, 1, 2.5, 5 and 7,5µM), zanubrutinib (1, 2, 5, 10 and 15µM), dasatinib (0.02, 0.04, 0.1, 0.2and 0,3 µM) and idelalisib (0.5, 1, 2.5, 5 and 7,5µM), either alone or in combination with I3C, as indicated in the Fig. 13. After 48 hours, the percentage of viable cells was determined by a colorimetric assay considering the control value as 100% viability (left). Plots at the right show the graphic representation of the combination index (CI) for the fraction affected (Fa) for each treatment group, calculated with the Calcusyn software.

## Claims

1. A pharmaceutical combination comprising:
c) a compound of formula (I) 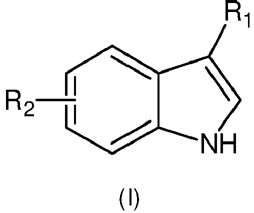 or its derivatives, pharmaceutical acceptable salts or solvates, wherein
R¹ represents an atom other than hydrogen, of electronegative nature selected from -NH₂ , -OH, -SH, -PH₂ , -CI, -Br, -F, -I, -OR, -NR₂ , -SR, - PR₂ , -CH₂OH, -CH₂NH₂ , -CH₂SH, - CH₂PH ₂ , -CH₂Cl, -CH₂Br, -CH₂F and -CH₂I, and R² represents a substituent in position 5 or 6 of the indole ring selected from hydrogen, an alkyl group, a halogen atom selected from chlorine, bromine, fluorine or iodine, or an electronegative atom linked to alkyl chains selected from -OR, -NR₂, -SR and PR₂;
where each R is independently an alkyl group, linear or branched and
d) an inhibitor of the BCR pathway selected from ibrutinib, acalabrutinib, zanubrutinib, evobrutinib, tirabrutinib, vecabrutinib, dasatinib, idelalisib, copanlisib, duvelisib, gedatolisib, buparlisib, alpelisib, taselisib or rigosertib.

2. The pharmaceutical combination of claim 1 wherein the derivative of the compound of formula (I) is a compound of formula (II): 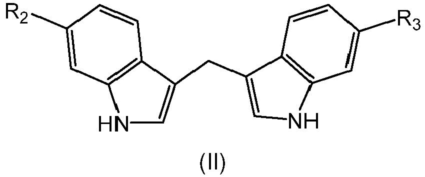 wherein R₂ and R₃ are the same or different substituents and represent hydrogen, an alkyl group, a halogen atom selected from chlorine, bromine, fluorine or iodine, or an electronegative atom attached to alkyl chains selected from -OR, -NR₂, -SR and PR₂, where each R is independently an alkyl group, linear or branched.

3. The pharmaceutical combination of claim 1 wherein the derivative of the compound of formula (I) is a compound of formula (III): 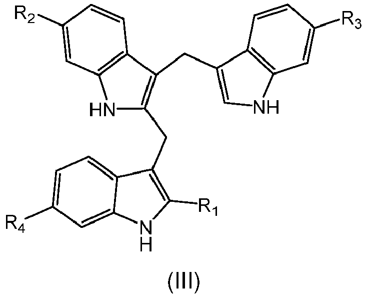 wherein R₁ is as defined in claim 1 and, R₂, R₃ and R₄ are the same or different substituents and represent hydrogen, an alkyl group, a halogen atom selected from chlorine, bromine, fluorine or iodine, or an electronegative atom attached to alkyl chains selected from -OR, -NR₂, -SR and PR₂, where each R is independently an alkyl group, linear or branched.

4. The pharmaceutical combination of any one of claims 1 to 3 wherein the compound of formula (I) is selected from the following compounds:

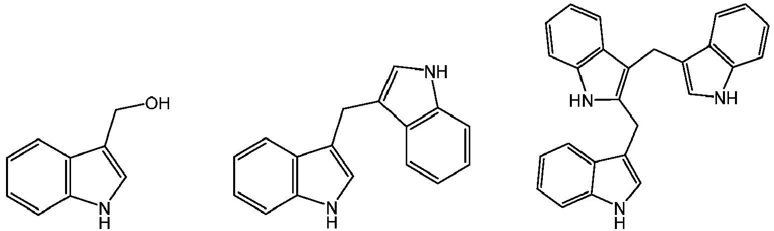

5. The pharmaceutical combination of any one of claims 1 to 4 wherein the inhibitor or the BCR pathway is ibrutinib.

6. The pharmaceutical combination of any one of claims 1 to 5 wherein the compound of formula (I) is indole-3-carbinol and the inhibitor of the BCR pathway is ibrutinib.

7. The pharmaceutical combination of any one of claims 1 to 6 which comprises: c) a further active principle which is selected from fludarabine or cladribine.

8. A pharmaceutical combination according to any one of claims 1 to 7 for use in the treatment of B lymphoid neoplasms, preferably selected from chronic lymphocytic leukaemia, small cell lymphocytic lymphoma, Burkitt's lymphoma, diffuse large B-cell lymphoma, lymphoplasmacytic lymphoma, mantle cells lymphoma, Waldenstrom's macroglobulinemia, diffuse large B-cell lymphoma, follicular lymphoma, marginal zone lymphoma or multiple myeloma.

9. The pharmaceutical combination for use according to claim 8 wherein the use of components (a), (b) and (c) is simultaneous, separate or sequential.

10. The pharmaceutical combination for use according to any one of claims 8 to 9 wherein the use of components (a), (b) and (c) is by oral or intravenous administration.

11. A pharmaceutical composition comprising the pharmaceutical combination according to any one of claims 1 to 7 and at least one acceptable carrier.

12. A pharmaceutical composition according to claim 11 for use in the treatment of B lymphoid neoplasms, preferably selected from chronic lymphocytic leukaemia, small cell lymphocytic lymphoma, Burkitt's lymphoma, diffuse large B-cell lymphoma, lymphoplasmacytic lymphoma, mantle cells lymphoma, Waldenstrom's macroglobulinemia, diffuse large B-cell lymphoma, follicular lymphoma, marginal zone lymphoma or multiple myeloma.

13. The pharmaceutical composition for use according to claim 12 wherein the composition is used by oral or intravenous administration.

14. The pharmaceutical composition for use according to any one of claims 12 or 13 which comprises a further active principle selected from fludarabine or cladribine.
